# EUROPEAN PATENT SPECIFICATION

(11) **EP 1 167 331 B1**
(45) Date of publication and mention of the grant of the patent: **23.08.2006**
(21) Application number: 01114937.4
(22) Date of filing: 20.06.2001
(51) Int. Cl.: C07C 29/56, C07C 33/042

(54) **Isomerization of a pentol**
Isomerisierung eines Pentols
Isomérisation d'un pentol

(30) Priority: 30.06.2000 EP 00113884
(43) Date of publication of application: 02.01.2002
(73) Proprietor: DSM IP Assets B.V., 6411 TE Heerlen (NL)
(72) Inventor: Gloor, Arnold, 4053 Basle (CH); Gruendler, Hansjoerg, 4310 Rheinfelden (CH); Simon, Werner, 4125 Riehen (CH)
(74) Representative: Müller, Ingrid

(56) References cited:
- EP-A- 0 621 254
- DATABASE CA [Online] CHEMICAL ABSTRACTS SERVICE, COLUMBUS, OHIO, US; SKODA, ALOJZ ET AL: "Allylic rearrangement of 3-methyl-1-penten-4-yn-3-ol to cis-3-methyl-2-penten-4-yn-3-ol catalyzed by a cation-exchange resin" retrieved from STN Database accession no. 114:228365 CA XP002233971 & CS 269 025 B (CZECH.) 11 April 1990 (1990-04-11)
- DATABASE CA [Online] CHEMICAL ABSTRACTS SERVICE, COLUMBUS, OHIO, US; SAMOKHVALOV, G. I. ET AL: "Trans-3-Methyl-2-penten-4-yn-1-ol" retrieved from STN Database accession no. 82:42969 CA XP002233972 & SU 445 637 T (ALL-UNION SCIENTIFIC-RESEARCH INSTITUTE OF VITAMINS) 5 October 1974 (1974-10-05)
- DATABASE WPI Section Ch, Week 199207 Derwent Publications Ltd., London, GB; Class E17, AN 1992-051995 XP002233973 & JP 03 294245 A (NIPPON OILS & FATS CO LTD), 25 December 1991 (1991-12-25)

## Description

The present invention concerns a process for the catalytic isomerization of Z-3-methylpent-2-en-4-yn-1-ol to E-3-methylpent-2-en-4-yn-1-ol, hereinafter referred to for brevity as the isomerization of "Z-pentol" to "E-pentol".

The known acid-catalysed allylic rearrangement of 3-methylpent-1-en-4-yn-3-ol affords in thermodynamic equilibrium an isomeric mixture of Z- and E-pentol in the ratio Z-: E-pentol of about 85 : 15. These stereoisomers can if desired be separated from each other by physical means, e.g. by fractional distillation, to afford each stereoisomer in relatively good purity. The stereoisomer produced and isolated in the larger proportion, i.e. Z-pentol, is a useful intermediate, e.g. for the manufacture of vitamin A, and the stereoisomer produced and isolated in the smaller proportion, i.e. E-pentol, is also a useful intermediate, in this case e.g. for the manufacture of astaxanthin, zeaxanthin and further carotenoids. The situation may be schematically illustrated as follows, whereby the formulae are presented by conventional line representation:

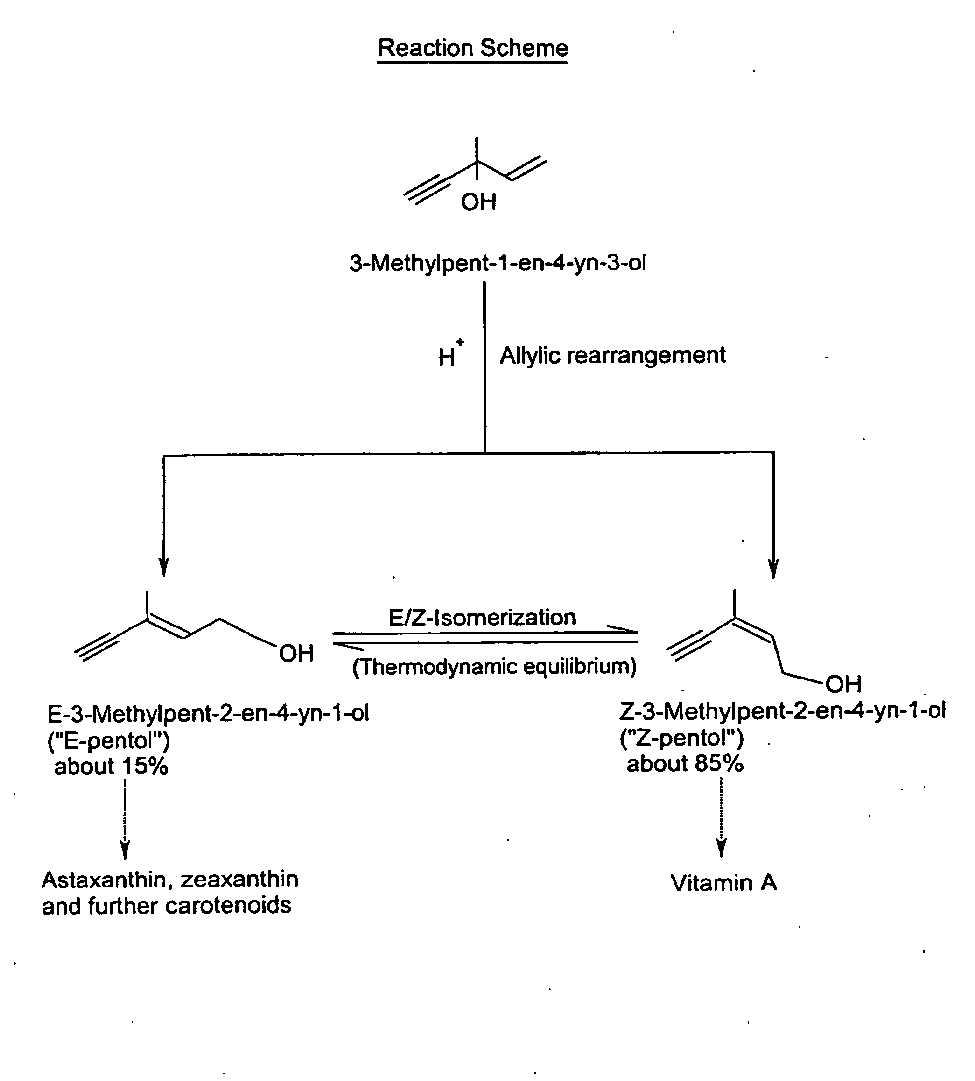

According to the relative requirement for the one or the other stereoisomer depending on the relative amounts of the carotenoid and vitamin A end products to be produced therefrom there exists an economical need to shift the above equilibrium of E- and Z-pentol from the thermodynamic one and accordingly to influence the stereoisomeric ratio of these two useful intermediates; it is seldom economically feasible to separate the stereoisomers from a mixture in the thermodynamic equilibrium (about 85 : 15 Z- : E-pentol) as above. Indeed, since Z-pentol is the thermodynamically more stable pentol product a shifting of the equilibrium in the direction Z- --> E- entails an input of energy which would be justified if the relative requirement for astaxanthin, zeaxanthin and further carotenoids significantly exceeds about 15% of the total of both isomers. In this case, for example, there exists a need for a process for isomerizing a mixture of Z- and E-pentol, e.g. one in thermodynamic equilibrium with a Z- : E- ratio of about 85 : 15, to one with an increased proportion, i.e. higher than about 15%, of E-pentol. This need has been surprisingly achieved by the catalytic isomerization process of the present invention which involves the use of bromine radicals (Br•) as the catalyst for isomerizing Z-pentol to E-pentol in a mixture of both these stereoisomers.

SU 445 637 discloses the isomerization of a Z- to E-3-methylpent-2-en-4-yn-1-ol by using group IB or IIB metal salts or complexes.

According to the present invention there is provided a process for catalytically isomerizing Z-pentol to E-pentol in a mixture of both these stereoisomers, which process is characterized in that as the isomerization catalyst there are employed bromine radicals (Br•) and the isomerization is conducted in a two-phase system of an aqueous phase and the pentol phase, the stereoisomeric pentol mixture optionally being present in a water-immiscible organic solvent, with intermixing of the reaction mixture and at temperatures from -10°C to 100°C.

In principle any chemical system for generating the bromine radicals necessary for the performance of the catalytic isomerization process of the present invention may be utilized in said process, and each such chemical system gives rise to a particular embodiment of the process.

Common to all the chemical systems for generating the bromine radicals is the actual source of bromine radicals, which is suitably an alkali metal or alkaline earth metal bromide, or ammonium bromide. As the alkali metal or alkaline earth metal bromide there comes into consideration particularly sodium or potassium bromide or, respectively, calcium or magnesium bromide. Preferably sodium bromide or potassium bromide is employed as the source of the bromine radicals.

The amount in moles of such bromide salt employed relative to the amount of pentol starting material (mixture of Z- and E-pentols) in moles is suitably 0.2 mole to 5 moles / mole, preferably 0.2 mole to 1 mole / mole, most preferably 0.2 mole to 0.5 mole / mole.

In one embodiment of the process of the present invention a salt of a heavy metal is used as the catalyst for promoting the generation of bromine radicals from the source thereof, and in addition oxygen is generally used as an auxiliary agent for promoting the bromine radical generation. Examples of the heavy metals (cationic constituents) of these salts are titanium, vanadium, chromium, manganese, cobalt, nickel, zirconium, niobium, praseodymium, hafnium and lead, and examples of the anionic constituents of these salts are chloride, bromide, oxide, sulphate, oxychloride (OCl₂⁴⁻) and acetate. Specific examples of such heavy metal salts are titanous chloride (TiCl₃), vanadium trichloride (VCl₃), vanadium dioxide (V₂O₄), vanadium pentoxide (V₂O₅), chromic chloride (CrCl₃), manganous bromide (MnBr₂), manganese dioxide (MnO₂), manganous sulphate (MnSO₄), manganous acetate [Mn(OCOCH₃)₂], manganic acetate [Mn(OCOCH₃)₃], cobaltous bromide (CoBr₂), nickelous bromide (NiBr₂), zirconic oxychloride (ZrOCl₂) niobium pentoxide (Nb₂O₅), praseodymium chloride (PrCl₃), praseodymium oxide (Pr₆O₁₁), hafnium tetrachloride (HfCl₄) and plumbous bromide (PbBr₂). A heavy metal bromide is preferably used as the catalyst. Independently of the nature of the anion, manganese, especially manganous (Mn²⁺) salts, are preferably used as the catalysts.

The amount in moles of the heavy metal salt employed relative to the amount of pentol starting material (mixture of Z- and E-pentols) is suitably 0.001 mole to 0.5 mole / mole, preferably 0.001 to 0.3 mole / mole, most preferably 0.01 to 0.03 mole / mole.

As indicated above, oxygen is also generally used in the promotion of the bromine radical generation. As will be evident from the nature of the heavy metal salts, various oxidation potentials (levels) are represented by the metal ions in said salts, from as low as 2+,. e.g. manganese(II) (manganous) in MnBr₂, MnSO₄ and Mn(OCOCH₃)₂, to as high as 4+, e.g. manganese(IV) in MnO₂, or even 5+, e.g. vanadium(V) and niobium(V) in V₂O₅ and Nb₂O₅, respectively. The function of the oxygen, if used, is to raise the oxidation level of the heavy metal cations to render them effective in generating the bromine radicals from the bromide anions present. Thus a relatively low concentration of heavy metal cations with a high oxidation level suffices to generate the bromine radicals. For example, Mn²⁺ ions can be elevated to Mn³⁺ ions with oxygen , and a relatively small amount of such Mn³⁺ ions enables the bromine radicals to be generated. Indeed, if heavy metal cations of a sufficiently high oxidation level are present at the outset, the presence of oxygen can even be dispensed with. As a further example, Mn²⁺ ions are not able to generate bromine radicals from the bromide in the absence of oxygen, but Mn³⁺ ions can do this.

In those cases where oxygen is used as an auxiliary agent for the bromine radical generation, it can be used as such or in admixture with an inert gaseous component, e.g. with nitrogen in air. The oxygen gas or gas mixture, preferably containing at least 5 vol. % of oxygen, is suitably continuously passed through the two-phase reaction medium during the isomerization process. The rate of oxygen or oxygen mixture passage is suitably 5 l/h to 200 1/h, preferably 20 l/h to 50 1/h. The technical means of oxygen passage is unimportant and can be conventional technical methodology, such as the use of a stirrer with jet outlets through which the oxygen is passed and released continuously into the stirred reaction medium. The oxygen gas or gas mixture can be used under pressure, suitably at a pressure up to a maximum of 50 bar (5 MPa), which serves to accelerate the isomerization.

In the process of the present invention the mixture of pentol stereoisomers may form the pentol phase, or the stereoisomers may be dissolved in an essentially water-immiscible organic solvent. Such solvent is suitably an alkane, e.g. pentane, hexane or heptane; an aromatic hydrocarbon, e.g. benzene or toluene; a chlorinated alkane, e.g. methylene chloride, chloroform or carbon tetrachloride; or an aliphatic ether, e.g. diethyl ether or diisopropyl ether. The aqueous phase serves to dissolve the alkali metal, alkaline earth metal or ammonium bromide, i.e. the source of the bromine radicals, and also the heavy metal salt, and is water alone or can be an aqueous methanolic solution.

The catalytic isomerization process according to this first embodiment of the process is conveniently effected in a pH range from 0 to 2.5. It has been established that on conducting the process at higher pH values, e.g. from pH 2.5 to pH 4.0, the yield of the desired E-pentol is increasingly reduced as the pH value is increased. The preferred pH range is from 0.5 to 1.

To adjust the pH value as above the presence of a strong mineral acid, i.e. one with a pKa value of less than about 2, or of the organic acid acetic acid in the reaction medium is required. For this purpose there may suitably be used hydrochloric, hydrobromic, sulphuric, nitric or perchloric acid as the mineral acid, or , as mentioned above, acetic acid, the chosen acid being added in sufficient quantity, also if necessary during the initiated isomerization process, to bring or maintain the pH within the above range. If acetic acid is used, this is preferably approx. 50% aqueous acetic acid. Preferably hydrobromic acid is used as the strong mineral acid.

As mentioned above the catalytic isomerization process of the present invention is effected at temperatures from -10°C to 100°C. If the first embodiment is used, the temperature range for both a batch and a continuous methodology is more suitably from 0°C to 70°C, and in the case of a continuous methodology the temperature may even be suitably raised for short residence times of a few minutes to about 90°C. Preferably the temperature is from 40°C to 60°C.

The isomerization process according to the first embodiment can be conducted using conventional procedural methodology. One suitable procedure involves heating a mixture of the bromide salt, the heavy metal salt and the acid required for pH adjustment in water to the desired reaction temperature under intensive mixing, e.g. through stirring, and then adding the mixture of pentol stereoisomers, as such or in solution in the essentially water-immiscible organic solvent, and also starting the oxygen passage. Intensive stirring is continued during the isomerization.

After completion of the isomerization process there generally results a two-phase mixture of which the aqueous and the organic phases can be separated by conventional means. The aqueous phase contains essentially the heavy metal salt catalyst, the bromide salt and the acid, and can be reused if desired for further isomerization reactions with a new Z- and E-pentol mixture; if necessary additional bromide salt is added and/or the pH is adjusted by addition of more acid. The organic phase contains essentially as the dissolved material the Z/E-isomeric mixture of increased E-isomer content compared with that of the starting pentol mixture. It can be washed with water to neutrality by conventional means, and the pure E- and Z-isomers can be isolated therefrom for example by fractional distillation. The isolated E- and Z-pentols can then be used as desired, especially for the production of astaxanthin, zeaxanthin and further carotenoids and, respectively, for the production of vitamin A.

In a further embodiment of the process of the present invention there is used as the catalyst for promoting the generation of the bromine radicals a strong peroxide-type oxidizing agent. More particularly, such catalyst is an alkali metal or alkaline earth metal peroxomonosulphate, peroxoborate, peroxodisulphate or peroxodiphosphate, or the system hydrogen peroxide/alkali metal or alkaline earth metal sulphate. In each case the alkali metal is suitably sodium or potassium, and the alkaline earth metal suitably calcium or magnesium. Examples of these catalysts are potassium peroxomonosulphate, sodium peroxoborate, sodium peroxodisulphate, potassium peroxodisulphate, potassium peroxodiphosphate and hydrogen peroxide/sodium sulphate. The catalyst is preferably a peroxodisulphate or the system hydrogen peroxide/alkali metal or alkaline earth metal sulphate, most preferably the latter catalyst system.

The amount in moles of strong peroxide-type oxidizing agent (catalyst) used for the isomerization reaction relative to the amount of pentol starting material is suitably 0.01 to 0.5 mole / mole, preferably 0.015 to 0.2 mole / mole. In the case of the hydrogen peroxide/sulphate catalyst system the hydrogen peroxide is conveniently used in aqueous solution, preferably of concentration 30%, and the amount of sulphate salt employed is conveniently 0.1 to 50 mole % of the molar amount of pentol starting material.

In contrast to the first embodiment described above, the present embodiment does not require oxygen as an auxiliary agent for promoting the generation of the bromine radicals. Indeed, this further embodiment can be effected in an inert atmosphere, e.g. nitrogen or argon.

In the present embodiment the isomerization process is conveniently carried out in a two-phase medium in which the aqueous phase contains essentially the dissolved alkali metal, alkaline earth metal or ammonium bromide, i.e. the source of the bromine radicals, and optionally also added acid for any necessary pH adjustment, and the organic phase is formed from the mixture of pentol stereoisomers, which may optionally be dissolved in an organic solvent. Said organic solvent is suitably a chlorinated alkane, e.g. methylene chloride, chloroform or carbon tetrachloride; a lower, especially C₁₋₆-, alkanol, e.g. methanol, ethanol, isopropanol, n-butanol or tert. butanol; an aliphatic ketone, e.g. isobutyl methyl ketone; an aliphatic ester, e.g. ethyl acetate; acetonitrile; an organic carbonate, e.g. dimethyl carbonate; an alicyclic hydrocarbon, e.g. methylcyclohexane; or an aromatic hydrocarbon, e.g. toluene. The use of an organic solvent in the reaction medium appears to reduce this tendency of the pentol stereoisomers to decompose and is also of advantage by facilitating the isolation of the product after the reaction.

Regardless of the use or not of an organic solvent to dissolve the mixture of pentol stereoisomers, the volume of water per mole of such pentol stereoisomer mixture is conveniently 50 ml to 800 ml of water / mole of pentol stereoisomer mixture, preferably 50 ml to 200 ml of water / mole of pentol stereoisomer mixture. If a low volume of water is used, i.e. 50 - 100 ml, the temperature at which the isomerization reaction is conducted is suitably somewhat higher than if volumes above about 100 ml are used in order to compensate for the lower heat capacity of the reaction mixture.

The catalytic isomerization process according to this further embodiment, and in contrast to the first embodiment, is less influenced by the pH of the reaction medium, and indeed can generally be effected in the broad pH range of 0 to 10; accordingly, the addition of acid to the medium for adjustment of the pH is usually unnecessary. The preferred pH range is however from 0 to 7. By conducting the isomerization reaction in the neutral pH range, i.e. around pH 7, any partial decomposition of the pentol stereoisomers, which occurs to some extent in the acid pH range (< pH 7), is considerably reduced. If pH adjustment is effected, the same kind of acid may be added to the reaction medium as given for the previous embodiment.

The catalytic isomerization process of this further embodiment for both a batch and a continuous methodology is suitably effected at temperatures from -10°C to 70°C; in the case of a continuous methodology the temperature may even be raised for short residence times of a few minutes to about 100°C, whereby the tendency of the pentol stereoisomers to decompose at such higher temperatures must be observed by not prolonging unnecessarily the heating in the upper temperature range. The catalytic isomerization process is preferably effected at temperatures from 40°C to 60°C.

The isomerization process according to this further embodiment too can be conducted using conventional procedural methodology. An especially suitable methodology comprises heating the two-phase medium consisting of the mixture of pentol stereoisomers, water, the bromide salt and any acid required for pH adjustment to the desired reaction temperature under gasification with an inert gas, such as nitrogen or argon, and under intensive mixing, e.g. through stirring, and then adding the catalyst as a crystalline solid or in aqueous solution. In the case of using the system hydrogen peroxide/alkali metal or alkaline earth metal sulphate as the catalyst the above especially suitable methodology differs in that the sulphate is included in the two-phase medium for heating under gasification and intensive mixing to the desired reaction temperature, and then the hydrogen peroxide in aqueous solution is added. In all cases the reaction mixture is suitably mixed further, e.g. by stirring, and if necessary the pH adjusted periodically, until it has been established that the isomerization process has proceeded to a more or less constant isomerization equilibrium. Thereafter the mixture is suitably cooled, preferably to room temperature or thereabouts, and the isolation of the product effected.

After completion of the isomerization process according to this further embodiment there generally results a two-phase mixture with in certain cases a solid residue consisting of the insoluble salts, such as various sulphates, hydrogen sulphates etc. Any solid constituents can be readily removed, e.g. by filtration, and the remaining two-phase liquid medium containing the Z/E-isomeric mixture of pentols with increased E-isomer content in the organic phase and an aqueous phase containing remaining dissolved salts then treated essentially as described above in connection with the final isolation procedure of the first embodiment to afford the isolated E- and Z-pentols. In this case, too, the aqueous phase containing dissolved salts, or the salts isolated therefrom, can be reused if desired for further isomerization reactions with a new Z- and E-pentol mixture.

Regardless of the embodiment employed, the reaction duration to the achievement of the isomerization equilibrium depends very much on the particular reaction conditions employed and can amount to a few minutes to several hours. As an example, in certain instances of the isomerization process being conducted at 85°C using the two-phase solvent system water and methylene chloride the isomerization equilibrum is rapidly achieved, i.e. within about 2 minutes. In any event, such reaction conditions as the concentration of the bromide salt and the employed amount of catalyst exert a strong influence on the reaction duration.

The process in accordance with the invention is illustrated by the following Examples:

### Example 1

149.55 g of potassium bromide, 5.85 g of manganous acetate tetrahydrate, 250 ml of water and 200 ml of glacial acetic acid were introduced successively into a reactor and the mixture was then stirred and warmed to 50°C. Air was led through the resulting stirred solution at a rate of 20 l/h, and 108.54 g of (mainly) Z-3-methylpent-2-en-4-yn-1-ol [98.7% of the Z-isomer and 1.0% of the E-isomer according to gas chromatography (GC)] were added dropwise over 10 minutes.

After a reaction duration of 1 hour the mixture was cooled down to room temperature and with portionwise addition of 500 ml of water and 500 ml of methylene chloride extracted into the organic phase in each case. The combined organic phases were concentrated at 30°C under reduced pressure, yielding 176.16 g of a dark brown liquid. According to GC said liquid contained 47.4% of Z-3-methylpent-2-en-4-yn-1-ol and 5.9% of the E-isomer. This corresponds to a yield of 8.7% of E-3-methylpent-2-en-4-yn-1-ol from the initially available Z-isomer.

### Example 2

Low oxygen air (5 vol. % O₂ and 95 vol. % N₂) was introduced at a rate of 201/h through the sintered glass filter of a double-walled reactor fitted with a sintered glass base, followed by 99.7 g of potassium bromide, 2.72 g of manganous sulphate monohydrate and 300 ml of 0.25N sulphuric acid, and the mixture was stirred and warmed to 70°C. Then the rate of introduction of the low oxygen air was increased to 50 l/h, and 72.36 g of (mainly) Z-3-methylpent-2-en-4-yn-1-ol (composition as in Example 1) were introduced over 10 minutes. After a stirring period of 1 hour at 70°C the introduction of low oxygen air was stopped and the resulting two-phase reaction mixture was released from the reactor through the sintered glass filter via the exit conduit at the base.

The dark brown organic phase of the released reaction mixture was separated from the aqueous phase and washed with water, and the aqueous phase was washed with 30 ml of diisopropyl ether. A GC analysis of the combined organic and diisopropyl ether phases indicated a yield 11.8% of E-3-methylpent-2-en-4-yn-1-ol from the initially available Z-isomer.

### Example 3

450 ml of water, 149.55 g of potassium bromide and 6.99 g of manganous bromide tetrahydrate were introduced successively into a double-walled reactor and the mixture was stirred and heated to 50°C. After adjustment of the pH to 1.0 with 6.0 ml of 30% hydrobromic acid air was led through the mixture at a rate of 20 l/h and 109.11 g of (mainly) Z-3-methylpent-2-en-4-yn-1-ol (98.6% of the Z-isomer and 0.6% of the E-isomer according to GC) were added portionwise over 10 minutes. After an hour's stirring and air passage at 50°C the mixture was cooled to 25°C and the two phases separated from each other.

The organic phase was washed with 45 ml of water, and the aqueous phase extracted with 45 ml of diisopropyl ether. A GC analysis of the combined organic and diisopropyl ether phases indicated a yield of 13.5% of E-3-methylpent-2-en-4-yn-1-ol from the initially available Z-isomer.

### Example 4

149.55 g of potassium bromide, 2.28 g of manganese dioxide and 450 ml of 0.25N sulphuric acid were introduced successively into a double-walled reactor and the mixture was then stirred and warmed to 50°C. 4.5 ml of 62% hydrobromic acid were added to the mixture for fully dissolving the manganese dioxide, whereupon the pH of the solution was 0.8, and within 15 minutes thereafter 121.77 g of (mainly) Z-3-methylpent-2-en-4-yn-1-ol (containing water; 87.8% of the Z-isomer and 1.1% of the E-isomer according to GC) were added portionwise. After an hour's stirring at 50°C the mixture was cooled to 25°C and the two phases separated from each other.

The dark brown organic phase was washed with 45 ml of water and the aqueous phase was extracted with 45 ml of diisopropyl ether. A GC analysis of the combined organic and diisopropyl ether phases indicated a yield of 6.0% of E-3-methylpent-2-en-4-yn-1-ol from the initially available Z-isomer.

### Example 5

Under an atmosphere of argon 196 g of (mainly) Z-3-methylpent-2-en-4-yn-1-ol (about 98% of the Z-isomer and about 1% of the E-isomer according to GC) 244.3 g of potassium bromide and 703.5 g of 0.25N sulphuric acid were introduced successively into a double-walled reactor and the mixture was then stirred and warmed to 50°C. Then 50 g of manganic acetate dihydrate were added as rapidly as possible, causing the pH of the reaction mixture to rise from 0.5 to 3.7. Addition of 50% sulphuric acid adjusted the pH to 0.9, at which pH value the reaction was continued for the next 3 hours. After this reaction period and subsequent cooling to room temperature a GC analysis was effected which indicated that the yield of E-3-methylpent-2-en-4-yn-1-ol, taken from both liquid phases of the mixture, from the initially available Z-isomer amounted to 12.9%.

### Example 6

Under an atmosphere of argon 303.75 g of potassium bromide, 435 ml of 0.135N hydrobromic acid, 109.11g of (mainly) Z-3-methylpent-2-en-4-yn-1-ol (composition as in Example 3) and 10 ml of water were introduced successively into a double-walled reactor and the mixture was then stirred and warmed to 50°C. Then 16.2 g of potassium peroxodisulphate were added in one portion, and the reaction mixture was stirred for a further 15 minutes at 50°C, after which it was cooled to 20°C.

The solid salts which had precipitated out of the mixture on standing at 20°C were filtered off under reduced pressure and the two liquid phases separated from each other. The organic phase was washed with 45 ml of water, and the aqueous phase together with the precipitated salts were extracted with 50 ml of diisopropyl ether. A GC analysis of the combined organic and diisopropyl ether phases indicated a yield of 13.5% of E-3-methylpent-2-en-4-yn-1-ol from the initially available Z-isomer.

### Example 7

Under an atmosphere of argon 147.96 of potassium bromide, 424 ml of 0.135N hydrobromic acid, 106.71 g of (mainly) Z-3-methylpent-2-en-4-yn-1-ol (97,6% of the Z-isomer and 1.1% of the E-isomer according to GC) and 10 ml of water were introduced successively into a double-walled reactor and the mixture was then stirred and warmed to 50°C. Then 24.77 g of potassium peroxodiphosphate were added in one portion, which raised the pH of the reaction mixture from 0.6 to 5.4. Using 30% hydrobromic acid the pH was adjusted to 1.0 and with the use of a pH-stat said value was maintained during the whole 4 hours duration of the isomerization reaction. After this reaction period the mixture was cooled to 25°C.

The two liquid phases were then separated from each other. The organic phase was washed with 45ml of water, and the aqueous phase was extracted with 45 ml of diisopropyl ether. A GC analysis of the combined organic and diisopropyl ether phases indicated a yield of 7.0% of E-3-methylpent-2-en-4-yn-1-ol from the initially available Z-isomer.

### Example 8

Under an atmosphere of argon 151.87 g of potassium bromide, 370 ml of 0.159N hydrobromic acid, 109.11 g of (mainly) Z-3-methylpent-2-en-4-yn-1-ol (composition as in Example 3) and 10 ml of water were introduced successively into a double-walled reactor and the mixture was then stirred and warmed to 50°C. Using a dosage pump a solution of 19.01 g of potassium peroxomonosulphate (47%) in 65 ml of water was added portionwise within 30 minutes to the reaction mixture at 50°C. There followed a further 3.5 hours reaction period, after which the mixture was cooled to 25°C.

The two liquid phases were separated from each other. The organic phase was washed with 45 ml of water, and the aqueous phase with 45 ml of diisopropyl ether. A GC analysis of the combined organic and diisopropyl ether phases indicated a yield of 3.6% of E-3-methylpent-2-en-4-yn-1-ol from the initially available Z-isomer.

### Example 9

Under an atmosphere of argon 29.21 g of sodium bromide, 7.57 g of sodium sulphate, 50 ml of water and 96.66 g of (mainly) Z-3-methylpent-2-en-4-yn-1-ol (98.5% of the Z-isomer and 0.9% of the E-isomer according to GC) were introduced successively into a sulphonation flask, and the mixture was warmed to 50°C with stirring. 10.4 ml of 30% hydrogen peroxide were then added dropwise within 15 minutes and the reaction mixture was stirred at 50°C for 4 hours. During this reaction time the pH of the mixture rose from 5.3 to 6.1.

Then the mixture was cooled to 20°C and the precipitated salts removed by filtration. A GC analysis of the two liquid phases indicated a yield of 14.3% of E-3-methylpent-2-en-4-yn-1-ol from the initially available Z-isomer.

### Example 10

Under an atmosphere of argon 262.63 g of sodium bromide, 250 ml of water and 109.53 g of (mainly) Z-3-methylpent-2-en-4-yn-1-ol (composition as in Example 7) were introduced successively into a double-walled reactor and the mixture was then stirred and warmed to 50°C. A solution of 14.12 g of sodium peroxodisulphate in 200 ml of water, previously submitted to a passage of argon, was added to the mixture in the reactor with intensive stirring, causing the pH to sink from 5.65 to 2.25. After a further 30 minutes stirring at 50°C the isomerization had been completed. A GC analysis of both the liquid phases indicated a yield of 14.3% of E-3-methylpent-2-en-4-yn-1-ol from the initially available Z-isomer.

### Example 11 - 24

Using an analogous procedure to those describe in Examples 1 - 4 various heavy metal salts (promoters of bromine radical generation) were employed and the resulting yields of Z---> E-isomer conversion quantified. Standard reaction conditions were employed in these experiments, as follows:

For 14.42 g (0.15 mole) of 3-methylpent-2-en-4-yn-1-ol (98.5% of the Z- isomer and 0.5% of the E-isomer according to GC) there were employed per mole of said starting material 0.021 mole of heavy metal salt and 1.117 mole of potassium bromide. Completing the reaction mixture were in each case 60 ml of 0.135 N hydrobromic acid. The rate of air passage was 20 l/h. At a reaction temperature of about 50°C the isomerization was allowed to proceed for 2 - 3 hours.

The nature of the promoter and the yield in each case are presented in the following Table:

**Table**

| Example | Promoter | Yield (%) |
|---|---|---|
| 11 | MnBr₂ | 13.1 |
| 12 | V₂O₄ | 10.7 |
| 13 | VCl₃ | 10.7 |
| 14 | Pr₆O₁₁ | 7.5 |
| 15 | PrCl₃ | 7.2 |
| 16 | CrCl₃ | 6.3 |
| 17 | NiBr₂ | 5.5 |
| 18 | V₂O₅ | 5.4 |
| 19 | ZrOCl₂ | 5.0 |
| 20 | CoBr₂ | 4.9 |
| 21 | TiCl₃ | 4.5 |
| 22 | PbBr₂ | 2.8 |
| 23 | HfCl₄ | 2.7 |
| 24 | Nb₂O₅ | 2.1 |

## Claims

1. A process for catalytically isomerizing Z-3-methylpent-2-en-4-yn-1-ol to E-3-methylpent-2-en-4-yn-1-ol in a mixture of both these stereoisomers, **characterized in that** as the isomerization catalyst there are employed bromine radicals and the isomerization is conducted in a two-phase system of an aqueous phase and the stereoisomeric mixture phase, the stereoisomeric mixture optionally being present in a water-immiscible organic solvent, with intermixing of the reaction mixture and at temperatures from -10°C to 100°C.

2. A process according to claim 1, **characterized in that** the source of the bromine radicals is an alkali metal or alkaline earth metal bromide, or ammonium bromide, preferably sodium or potassium bromide.

3. A process according to claim 2, **characterized in that** the amount in moles of the bromide employed relative to the amount of stereoisomeric mixture starting material is 0.2 to 5 moles / mole, preferably 0.2 to 1 mole / mole, most preferably 0.2 to 0.5 mole / mole.

4. A process according to any one of claims 1 to 3, **characterized in that** a salt of a heavy metal is used as the catalyst for promoting the generation of bromine radicals from the source thereof, and in addition oxygen is optionally used as an auxiliary agent for promoting the bromine radical generation.

5. A process according to claim 4, **characterized in that** the heavy metal (cationic constituent) of the salt is selected from titanium, vanadium, chromium, manganese, cobalt, nickel, zirconium, niobium, praseodymium, hafnium and lead, and the anionic constituent of the salt is selected from chloride, bromide, oxide, sulphate, oxychloride and acetate.

6. A process according to claim 5, **characterized in that** the salt is titanous chloride (TiCl₃), vanadium trichloride (VCl₃), vanadium dioxide (V₂O₄), vanadium pentoxide (V₂O₅), chromic chloride (CrCl₃), manganous bromide (MnBr₂), manganese dioxide (MnO₂), manganous sulphate (MnSO₄), manganous acetate [Mn(OCOCH₃)₂], manganic acetate [Mn(OCOCH₃)₃], cobaltous bromide (CoBr₂), nickelous bromide (NiBr₂), zirconic oxychloride (ZrOCl₂), niobium pentoxide (Nb₂O₅), praseodymium chloride (PrCl₃), praseodymium oxide (Pr₆O₁₁), hafnium tetrachloride (HfCl₄) or plumbous bromide (PbBr₂).

7. A process according to any one of claims 4 to 6, **characterized in that** the amount in moles of the heavy metal salt employed relative to the amount of stereoisomeric mixture starting material is 0.001 mole to 0.5 mole / mole, preferably 0.001 to 0.3 mole / mole, most preferably 0.01 to 0.03 mole / mole.

8. A process according to any one of claims 4 to 7, **characterized in that** the oxygen is not used in those cases where the oxidation level of the heavy metal cation suffices to effect the generation of the bromine radicals from the bromide anions.

9. A process according to any one of claims 4 to 8, **characterized in that** the stereoisomeric mixture is dissolved in a water-immiscible organic solvent, said solvent being an alkane, e.g. pentane, hexane or heptane; an aromatic hydrocarbon, e.g. benzene or toluene; a chlorinated alkane, e.g. methylene chloride, chloroform or carbon tetrachloride; or an aliphatic ether, e.g. diethyl ether or diisopropyl ether, to afford the stereoisomeric mixture phase.

10. A process according to any one of claims 4 to 9, **characterized in that** it is effected in a pH range from 0 to 2.5, preferably from 0.5 to 1.

11. A process according to claim 1 or 2, **characterized in that** there is used as the catalyst for promoting the generation of the bromine radicals a strong peroxide-type oxidizing agent, especially an alkali metal or alkaline earth metal peroxomonosulphate, peroxoborate, peroxodisulphate or peroxodiphosphate, or the system hydrogen peroxide/alkali metal or alkaline earth metal sulphate, the alkali metal being sodium or potassium, and the alkaline earth metal being calcium or magnesium.

12. A process according to claim 11, **characterized in that** the catalyst is potassium peroxomonosulphate, sodium peroxoborate, sodium peroxodisulphate, potassium peroxdisulphate, potassium peroxodiphosphate or hydrogen peroxide/sodium sulphate.

13. A process according to claim 11 or 12, **characterized in that** the amount in moles of strong peroxide-type oxidizing agent (catalyst) employed relative to the amount of stereoisomeric mixture starting material is 0.01 to 0.5 mole / mole, preferably 0.015 to 0.2 mole / mole.

14. A process according to claim 11 or 12, **characterized in that** the catalyst is the system hydrogen peroxide/alkali metal or alkaline earth metal sulphate, the hydrogen peroxide being used in aqueous solution, preferably of concentration 30%, and the amount of sulphate salt employed being 0.1 to 50 mole% of the molar amount of stereoisomeric mixture starting material.

15. A process according to any one of claims 11 to 14, **characterized in that** the stereoisomeric mixture is dissolved in an organic solvent, said solvent being a chlorinated alkane, e.g. methylene chloride, chloroform or carbon tetrachloride; a C₁₋₆-, alkanol, e.g. methanol, ethanol, isopropanol, n-butanol or tert. butanol; an aliphatic ketone, e.g. isobutyl methyl ketone; an aliphatic ester, e.g. ethyl acetate; acetonitrile; an organic carbonate, e.g. dimethyl carbonate; an alicyclic hydrocarbon, e.g. methylcyclohexane; or an aromatic hydrocarbon, e.g. toluene, to afford the stereoisomeric mixture phase.

16. A process according to any one of claims 11 to 15, **characterized in that** the volume of water per mole of stereoisomeric mixture starting material is 50 ml to 800 ml of water, preferably 50 ml to 200 ml of water, per mole of stereoisomeric mixture.

17. A process according to any one of claims 11 to 16, **characterized in that** the two-phase medium consisting of the stereoisomeric mixture starting material, water, the bromide salt and any acid required for pH adjustment is heated to the desired reaction temperature under gasification with an inert gas, e.g. nitrogen or argon, and under intensive mixing, e.g. through strirring, and then the catalyst is added as a crystalline solid or in aqueous solution whereby in the case of using the system hydrogen peroxide / alkali metal or alkaline earth metal sulphate as the catalyst the sulphate is included in the two-phase medium for heating under gasification and intensive mixing to the desired reaction temperature, and the hydrogen peroxide in aqueous solution is then added.

## Patentansprüche

1. Verfahren zur katalytischen Isomerisierung von Z-3-Methylpent-2-en-4-in-1-ol zu E-3-Methylpent-2-en-4-in-1-ol in einem Gemisch dieser beiden Stereoisomere, **dadurch gekennzeichnet, daß** man als Isomerisierungskatalysator Bromradikale einsetzt und die Isomerisierung in einem Zweiphasensystem aus einer wäßrigen Phase und der Stereoisomerengemischphase, wobei das Stereoisomerengemisch gegebenenfalls in einem nicht mit Wasser mischbaren organischen Lösungsmittel vorliegt, unter Mischen der Reaktionsmischung und bei Temperaturen von -10°C bis 100°C durchführt.

2. Verfahren nach Anspruch 1, **dadurch gekennzeichnet, daß** es sich bei der Quelle der Bromradikale um ein Alkalimetall- oder Erdalkalimetallbromid oder Ammoniumbromid, vorzugsweise Natrium- oder Kaliumbromid, handelt.

3. Verfahren nach Anspruch 2, **dadurch gekennzeichnet, daß** die molare Menge des eingesetzten Bromids in Relation zur Menge an Stereoisomerengemisch-Edukt 0,2 bis 5 mol/mol, vorzugsweise 0,2 bis 1 mol/mol und ganz besonders bevorzugt 0,2 bis 0,5 mol/mol beträgt.

4. Verfahren nach einem der Ansprüche 1 bis 3,
**dadurch gekennzeichnet, daß** man als Katalysator zur Förderung der Erzeugung von Bromradikalen aus der Quelle dafür ein Schwermetallsalz verwendet und außerdem gegebenenfalls als Hilfsmittel für die Förderung der Bromradikalerzeugung Sauerstoff verwendet.

5. Verfahren nach Anspruch 4, **dadurch gekennzeichnet, daß** man das Schwermetall (den kationischen Bestandteil) des Salzes unter Titan, Vanadium, Chrom, Mangan, Cobalt, Nickel, Zirconium, Niob, Praseodym, Hafnium und Blei und den anionischen Bestandteil des Salzes unter Chlorid, Bromid, Oxid, Sulfat, Oxidchlorid und Acetat auswählt.

6. Verfahren nach Anspruch 5, **dadurch gekennzeichnet, daß** es sich bei dem Salz um Titan(III)-chlorid (TiCl₃), Vanadiumtrichlorid (VCl₃), Vanadiumdioxid (V₂O₄), Vanadiumpentoxid (V₂O₅), Chrom(III)-chlorid (CrCl₃), Mangan(II)-bromid (MnBr₂), Mangandioxid (MnO₂), Mangan(II)-sulfat (MnSO₄), Mangan(II)-acetat [Mn(OCOCH₃)₂], Mangan(III)-acetat [Mn(OCOCH₃)₃], Cobalt(II)-bromid (CoBr₂), Nickel(II)-bromid (NiBr₂), Zirconiumoxidchlorid (ZrOCl₂), Niobpentoxid (Nb₂O₅), Praseodymchlorid (PrCl₃), Praseodymoxid (Pr₆O₁₁), Hafniumtetrachlorid (HfCl₄) oder Blei(II)-bromid (PbBr₂) handelt.

7. Verfahren nach einem der Ansprüche 4 bis 6, **dadurch gekennzeichnet, daß** die molare Menge des eingesetzten Schwermetallsalzes in Relation zur Menge an Stereoisomerengemisch-Edukt 0,001 bis 0,5 mol/mol, vorzugsweise 0,001 bis 0,3 mol/mol und ganz besonders bevorzugt 0,01 bis 0,03 mol/mol beträgt.

8. Verfahren nach einem der Ansprüche 4 bis 7, **dadurch gekennzeichnet, daß** man in den Fällen keinen Sauerstoff verwendet, in denen die Oxidationsstufe des Schwermetallkations zur Erzeugung der Bromradikale aus den Bromidanionen ausreicht.

9. Verfahren nach einem der Ansprüche 4 bis 8, **dadurch gekennzeichnet, daß** man zur Bereitstellung der Stereoisomerengemischphase das Stereoisomerengemisch in einem nicht mit Wasser mischbaren organischen Lösungsmittel, bei dem es sich um ein Alkan, z.B. Pentan, Hexan oder Heptan, einen aromatischen Kohlenwasserstoff, z.B. Benzol oder Toluol, ein chloriertes Alkan, z.B. Methylenchlorid, Chloroform oder Tetrachlorkohlenstoff, oder einen aliphatischen Ether, z.B. Diethylether oder Diisopropylether, handelt, löst.

10. Verfahren nach einem der Ansprüche 4 bis 9, **dadurch gekennzeichnet, daß** man es in einem pH-Bereich von 0 bis 2,5 und vorzugsweise 0,5 bis 1 durchführt.

11. Verfahren nach Anspruch 1 oder 2, **dadurch gekennzeichnet, daß** man als Katalysator zur Förderung der Erzeugung der Bromradikale ein starkes Oxidationsmittel vom Peroxid-Typ, insbesondere ein Alkalimetall- oder Erdalkalimetallperoxomonosulfat, -peroxoborat, -peroxodisulfat oder -peroxodiphosphat, oder das System Wasserstoffperoxid/Alkalimetall- oder Erdalkalimetallsulfat verwendet, wobei es sich bei dem Alkalimetall um Natrium oder Kalium und bei dem Erdalkalimetall um Calcium oder Magnesium handelt.

12. Verfahren nach Anspruch 11, **dadurch gekennzeichnet, daß** es sich bei dem Katalysator um Kaliumperoxomonosulfat, Natriumperoxoborat, Natriumperoxodisulfat, Kaliumperoxodisulfat Kaliumperoxodiphosphat oder Wasserstoffperoxid/Natriumsulfat handelt.

13. Verfahren nach Anspruch 11 oder 12, **dadurch gekennzeichnet, daß** die molare Menge an eingesetztem starken Oxidationsmittel vom Peroxid-Typ (Katalysator) in Relation zur Menge an Stereoisomerengemisch-Edukt 0,01 bis 0,5 mol/mol und vorzugsweise 0,015 bis 0,2 mol/mol beträgt.

14. Verfahren nach Anspruch 11 oder 12, **dadurch gekennzeichnet, daß** es sich bei dem Katalysator um das System Wasserstoffperoxid/Alkalimetall- oder Erdalkalimetallsulfat handelt, wobei das Wasserstoffperoxid in wäßriger Lösung, vorzugsweise mit einer Konzentration von 30%, verwendet wird und die Menge an eingesetztem Sulfatsalz 0,1 bis 50 Mol-% der molaren Menge an Stereoisomerengemisch-Edukt beträgt.

15. Verfahren nach einem der Ansprüche 11 bis 14, **dadurch gekennzeichnet, daß** man zur Bereitstellung der Stereoisomerengemischphase das Stereoisomerengemisch in einem organischen Lösungsmittel, bei dem es sich um ein chloriertes Alkan, z.B. Methylenchlorid, Chloroform oder Tetrachlorkohlenstoff, ein C₁₋₆-Alkanol, z.B. Methanol, Ethanol, Isopropanol, n-Butanol oder tert.-Butanol, ein aliphatisches Keton, z.B. Isobutylmethylketon, einen aliphatischen Ester, z.B. Essigsäureethylester, Acetonitril, ein organisches Carbonat, z.B. Dimethylcarbonat, einen alicyclischen Kohlenwasserstoff, z.B. Methylcyclohexan, oder einen aromatischen Kohlenwasserstoff, z.B. Toluol, handelt, löst.

16. Verfahren nach einem der Ansprüche 11 bis 15, **dadurch gekennzeichnet, daß** das wasservolumen pro mol Stereoisomerengemisch-Edukt 50 ml bis 800 ml Wasser und vorzugsweise 50 ml bis 200 ml Wasser pro mol Stereoisomerengemisch beträgt.

17. Verfahren nach einem der Ansprüche 11 bis 16, **dadurch gekennzeichnet, daß** man das aus dem Stereoisomerengemisch-Edukt, Wasser, dem Bromidsalz und gegebenenfalls zur Einstellung des pH-Werts benötigter Säure bestehende zweiphasige Medium unter Begasung mit einem Inertgas, z.B. Stickstoff oder Argon, und intensivem Mischen, z.B. durch Rühren, auf die gewünschte Reaktionstemperatur erhitzt und dann den Katalysator in Form eines kristallinen Feststoffs oder in wäßriger Lösung zugibt, wobei man im Fall der Verwendung des Systems Wasserstoffperoxid/Alkalimetall- oder Erdalkalimetallsulfat als Katalysator das Sulfat dem zweiphasigen Medium zum Erhitzen auf die gewünschte Reaktionstemperatur unter Begasung und intensivem Mischen einverleibt und dann das Wasserstoffperoxid in wäßriger Lösung zugibt.

## Revendications

1. Procédé d'isomérisation catalytique du (Z)-3-méthylpent-2-èn-4-yn-1-ol en (E)-3-méthylpent-2-èn-4-yn-1-ol dans un mélange contenant ces deux stéréoisomères, **caractérisé en ce que** sont employés, au titre de catalyseur d'isomérisation, des radicaux brome, et **en ce que** l'isomérisation est menée dans un système biphasique comprenant une phase aqueuse et la phase du mélange des stéréoisomères, ledit mélange de stéréoisomères pouvant éventuellement être inclus dans un solvant organique non miscible à l'eau, ledit procédé impliquant la mise en contact du mélange réactionnel par agitation et se déroulant à des températures comprises entre -10 °C et 100 °C.

2. Procédé selon la revendication 1, **caractérisé en ce que** la source des radicaux brome est un bromure de métal alcalin ou de métal alcalino-terreux, ou du bromure d'ammonium, préférentiellement du bromure de sodium ou de potassium.

3. Procédé selon la revendication 2, **caractérisé en ce que** le rapport de la quantité du bromure employé en moles sur la quantité du mélange des stéréoisomères initial est compris entre 0,2 et 5 moles/mole, préférentiellement entre 0,2 et 1 mole/mole, le plus préférentiellement entre 0,2 et 0,5 mole/mole.

4. Procédé selon l'une quelconque des revendications 1 à 3, **caractérisé en ce qu'**un sel de métal lourd est employé au titre de catalyseur afin de promouvoir la formation de radicaux brome à partir de la source desdits radicaux, et **en ce que**, de plus, de l'oxygène peut éventuellement être utilisé en tant qu'agent auxiliaire dont le rôle est de favoriser la formation de radicaux brome.

5. Procédé selon la revendication 4, **caractérisé en ce que** le métal lourd (constituant cationique) du sel est sélectionné parmi le titane, le vanadium, le chrome, le manganèse, le cobalt, le nickel, le zirconium, le niobium, le praséodyme, l'hafnium et le plomb, et **en ce que** le constituant anionique du sel est sélectionné parmi le chlorure, le bromure, l'oxyde, le sulfate, l'oxychlorure et l'acétate.

6. Procédé selon la revendication 5, **caractérisé en ce que** le sel est le trichlorure de titane (TiCl₃), le trichlorure de vanadium (VCl₃), le dioxyde de vanadium (V₂O₄), le pentoxyde de vanadium (V₂O₅), le chlorure chromique (CrCl₃), le bromure manganeux (MnBr₂), le dioxyde de manganèse (MnO₂), le sulfate manganeux (MnSO₄), l'acétate manganeux [Mn (OCOCH₃)₂], l'acétate manganique [Mn(OCOCH₃)₃], le bromure cobalteux (CoBr₂), le bromure nickeleux (NiBr₂), l'oxychlorure zirconique (ZrOCl₂), le pentoxyde de niobium (Nb₂O₅), le chlorure de praséodyme (PrCl₃), l'oxyde de praséodyme (Pr₆O₁₁), le tétrachlorure d'hafnium (HfCl₄) ou le bromure plombeux (PbBr₂).

7. Procédé selon l'une quelconque des revendications 4 à 6, **caractérisé en ce que** le rapport de la quantité du sel de métal lourd employé en moles sur la quantité du mélange des stéréoisomères initial est compris entre 0,001 et 0,5 mole/mole, préférentiellement entre 0,001 et 0,3 mole/mole, le plus préférentiellement entre 0,01 et 0,03 mole/mole.

8. Procédé selon l'une quelconque des revendications 4 à 7, **caractérisé en ce que** l'oxygène n'est pas employé dans les cas où le niveau d'oxydation du cation de métal lourd suffit à la formation des radicaux brome à partir des anions bromure.

9. Procédé selon l'une quelconque des revendications 4 à 8, **caractérisé en ce que** le mélange de stéréoisomères est dissous dans un solvant organique non miscible à l'eau, ledit solvant étant un alcane, par exemple le pentane, l'hexane ou l'heptane ; un hydrocarbure aromatique, par exemple le benzène ou le toluène ; un alcane chloré, par exemple le dichlorométhane, le chloroforme ou le tétrachlorure de carbone ; ou un éther aliphatique, par exemple l'éther diéthylique ou l'éther diisopropylique, ceci constituant la phase de mélange de stéréoisomères.

10. Procédé selon l'une quelconque des revendications 4 à 9, **caractérisé en ce qu'**il est mené dans une gamme de pH comprise entre 0 et 2,5, préférentiellement entre 0,5 et 1.

11. Procédé selon la revendication 1 ou 2, **caractérisé en ce que** le catalyseur employé pour favoriser la formation des radicaux brome est un agent oxydant puissant de type peroxyde, en particulier un peroxomonosulfate, un peroxoborate, un peroxodisulfate ou un peroxodiphosphate d'un métal alcalin ou d'un métal alcalino-terreux, ou le système peroxyde d'hydrogène/sulfate de métal alcalin ou de métal alcalino-terreux, le métal alcalin étant le sodium ou le potassium, et le métal alcalino-terreux étant le calcium ou le magnésium.

12. Procédé selon la revendication 11, **caractérisé en ce que** le catalyseur est le peroxomonosulfate de potassium, le peroxoborate de sodium, le peroxodisulfate de sodium, le peroxodisulfate de potassium, le peroxodiphosphate de potassium ou le peroxyde d'hydrogène/sulfate de sodium.

13. Procédé selon la revendication 11 ou 12, **caractérisé en ce que** le rapport de la quantité d'agent oxydant puissant de type peroxyde (catalyseur) employé en moles sur la quantité de mélange de stéréoisomères initial est compris entre 0,01 et 0,5 mole/mole, préférentiellement entre 0,015 et 0,2 mole/mole.

14. Procédé selon la revendication 11 ou 12, **caractérisé en ce que** le catalyseur est le système peroxyde d'hydrogène/sulfate de métal alcalin ou de métal alcalino-terreux, le peroxyde d'hydrogène étant employé en solution aqueuse, préférentiellement de concentration égale à 30 %, et la quantité de sel de sulfate employée étant comprise entre 0,1 et 50 % en mole par rapport à la quantité de mélange de stéréoisomères initial.

15. Procédé selon l'une quelconque des revendications 11 à 14, **caractérisé en ce que** le mélange de stéréoisomères est dissous dans un solvant organique, ledit solvant étant un alcane chloré, par exemple le dichlorométhane, le chloroforme ou le tétrachlorure de carbone ; un alcanol en C₁-C₆, par exemple le méthanol, l'éthanol, l'isopropanol, le n-butanol ou le tert-butanol ; une cétone aliphatique, par exemple l'isobutylméthylcétone ; un ester aliphatique, par exemple l'acétate d'éthyle ; l'acétonitrile ; un carbonate organique, par exemple le carbonate de diméthyle ; un hydrocarbure alicyclique, par exemple le méthylcyclohexane ; ou un hydrocarbure aromatique, par exemple le toluène, ceci formant la phase de mélange de stéréoisomères.

16. Procédé selon l'une quelconque des revendications 11 à 15, **caractérisé en ce que** le volume d'eau par mole de mélange de stéréoisomères initial est compris entre 50 ml et 800 ml d'eau, préférentiellement entre 50 ml et 200 ml d'eau, par mole de mélange de stéréoisomères.

17. Procédé selon l'une quelconque des revendications 11 à 16, **caractérisé en ce que** le milieu biphasique constitué du mélange de stéréoisomères initial, d'eau, du bromure et de tout acide nécessaire à l'ajustement du pH est porté à la température de réaction voulue sous atmosphère de gaz inerte, par exemple d'azote ou d'argon, et en mélangeant vigoureusement, par exemple sous agitation, puis **en ce que** le catalyseur est ajouté sous forme de solide cristallin ou en solution aqueuse, sachant que dans le cas de l'emploi au titre de catalyseur du système peroxyde d'hydrogène/sulfate de métal alcalin ou de métal alcalino-terreux, le sulfate est incorporé dans le milieu biphasique lors de l'étape de chauffage sous atmosphère inerte et agitation vigoureuse jusqu'à atteindre la température de réaction désirée, avant ajout du peroxyde d'hydrogène en solution aqueuse.
